# EUROPEAN PATENT SPECIFICATION

(11) **EP 2 397 122 B1**
(45) Date of publication and mention of the grant of the patent: **14.05.2014**
(21) Application number: 11179637.1
(22) Date of filing: 16.06.2005
(51) Int. Cl.: A61K 9/20, A61K 31/13

(54) **Formulations of neramexane dosage forms**
Formulierungen aus Neramexan-Dosierformen
Formulations de formes pharmaceutiques de néramexane

(30) Priority: 17.06.2004 US 581244 P
(43) Date of publication of application: 21.12.2011
(62) Divisional of application: 05857458.3
(73) Proprietor: Merz Pharma GmbH & Co. KGaA, 60318 Frankfurt (DE)
(72) Inventor: Seiller, Erhard, 61130 Nidderau (DE); Hauptmeier, Bernhard, 63571 Gelnhausen (DE); Yang, Yan, Roslyn Heights New York 11577 (US); Janjikhel, Rajiv, South Setauket New York 11720 (US); Rao, Niranjan, Belle Mead, NJ 08502 (US); Periclou, Antonia, Rutherford, NJ 07070 (US); Abramowitz, Wattanporn, West Windsor, NJ 08550 (US); Dedhiya, Mahendra G., Pomona New York 10970 (US)
(74) Representative: Goddard, Christopher Robert

(56) References cited:
- WO-A-2005/044228
- US-A- 6 034 134
- US-A1- 2003 166 634

## Description

### FIELD OF THE INVENTION

The present invention is directed to pharmaceutical solid, oral dosage forms of compositions of 1-aminocyclohexane compounds which exhibit an immediate release profile, possess advantageous stability profiles and additionally disintegrate rapidly in aqueous solutions. The invention is particularly suitable for solid pharmaceutical dosage forms of 1-aminocyclohexane compounds in which a therapeutically effective amount of the active ingredient is available in the use environment shortly after administration. These compositions can be provided as dispersible tablets for administration as aqueous oral solution. The active ingredient is the 1-aminocyclohexane neramexane or an optical isomer, diastereomer, enantiomer, hydrate or pharmaceutically acceptable salt thereof.

### BACKGROUND OF THE INVENTION

1-Aminocyclohexanes, such as Memantine (1-amino-3,5-dimethyladamantane) and neramexane (1-amino-1,3,3,5,5-pentamethylcyclohexane), are moderate affinity, uncompetitive NMDA receptor antagonists with strong voltage dependency and rapid blocking/unblocking kinetics. Therefore, there is an existing and continual need in the art for solid oral formulations of 1-aminocyclohexane compounds, and more preferably memantine HCl (1-amino-3,5-dimethyladamantane hydrochloride) and neramexane mesylate (1-amino-1,3,3,5,5-pentamethylcyclohexane mesylate).

Solid oral drug compositions or preparations have various release profiles such as an immediate release profile as referenced by FDA guidelines ("Dissolution Testing of Immediate Release Solid Oral Dosage Forms", issued 8/1997, Section IV-A) or an extended release profile as referenced by FDA Guidelines ("Extended Release Oral Dosage Forms: Development, Evaluation, and Application of In Vitro/In Vivo Correlations", Food and Drug Administration, CDER, September 1997, Page 17). In the dissolution testing guideline for immediate release profiles, materials which dissolve at least 80% in the first 30 to 60 minutes in solution qualify as immediate release profiles. Therefore, immediate release solid dosage forms permit the release of most or all of the active ingredient over a short period of time, such as 60 minutes or less, and make rapid absorption of the drug possible. In contrast, extended release solid oral dosage forms permit the release of the active ingredient over an extended period of time in an effort to maintain therapeutically effective plasma levels over similarly extended time intervals, improve dosing compliance, and/or to modify other pharmacokinetic properties of the active ingredient.

U.S. Patent No. 5,382,601 provides solid pharmaceutical dosage forms containing memantine, which exhibit an extended two-phase release profile, with a portion of the drug being released immediately, followed by a sustained release of the remainder. The matrix of this formulation contains both a water-soluble and a water-insoluble salt of casein, preferably sodium and calcium caseinate. However, casein has an unpleasant taste; it is associated with the undesirable effect of exacerbating some side effects as disclosed in U.S. Patent No. 6,413,556; and displays instability in varying pH. Another concern regarding casein is the possibility of Bovine Spongiform Encephalitis (BSE) contamination or transmission of another infectious agent since casein is an animal-derived product.

A general method of preparing modified release N-methyl-D-aspartate (NMDA) receptor antagonists was described in U.S. Patent No. 6,194,000. This method involves preparing an instant release component and a modified release component to arrive at the final formulation. The patent discloses the formulations consisting of encapsulated beads previously coated using organic solvent-based systems. However, this patent does not specifically disclose compositions containing memantine or neramexane. The patent also does not teach how the release rates affect the Tₘₐₓ (time to maximum plasma concentration) or that this procedure will result in dose-proportional formulations.

Currently, a dosing regime of memantine of twice a day is employed using non-dose proportional immediate release tablets. After oral administration in man, memantine is completely absorbed (absolute bioavailability of approximately 100%). The time to maximum plasma concentrations (Tₘₐₓ) following oral doses of 10 to 40 mg memantine ranged between 3 and 7 hours, with peak plasma concentrations (Cₘₐₓ) after a single 20 mg oral dose ranging between 22 and 46 ng/mL. The AUC and Cₘₐₓ values of memantine increase proportionally with dose over the dosage range of 5 to 40 mg. The elimination half-life (T_{½}) of memantine is approximately 60-80 hours.

There is a need for dose-proportional memantine formulations which are readily achieved with immediate release formulations. Advantages of immediate release, dose-proportional formulations include improved ease of administration by allowing increases in dose without increasing the number of tablets that need to be administered, and increased flexibility in drug administration by allowing the target drug to be administered either as multiples of lower strength formulations or as one higher strength formulation. Another advantage of dose-proportional formulations of highly soluble and highly permeable drugs, particularly that of memantine and neramexane, is that the bioavailability of multiple strengths, e.g., 10 mg versus 80 mg, are considered identical and in accordance with the guidelines, "Waiver of In Vivo Bioavailability and Bioequivalence Studies for Immediate-Release Solid Oral Dosage Forms Based on a Biopharmaceutics Classification System", U.S. Department of Health and Human Services, Food and Drug Administration. Administration of increasing drug doses are often required as part of an up-titration regimen to the desired therapeutic dose because such regimens result in improved tolerability. In fact, current guidelines for use of memantine in the treatment of Alzheimer's Disease recommend that memantine be administered as a starting dose of 5 mg/day and escalated to the 20 mg/day dose by weekly increases in the dose by 5 mg. Dose proportional formulations are especially important for the treatment of diseases, such as neuropathic pain, which require up-titration to higher doses. The existence of dose proportional, immediate release formulations of different strengths of memantine ranging from 2.5 mg to 80 mg would therefore, allow ease and convenience in dosing during both the up-titration phase and during maintenance at the higher therapeutic dose levels.

### SUMMARY OF THE INVENTION

According to the present invention, it has now been found that 1-aminocyclohexanes, such as neramexane (1-amino-1,3,3,5,5-pentamethylcyclohexane), and their salts, including the hydrochloride, hydrobromide, mesylate salt as well as other pharmaceutically accepted salts, can be formulated into an immediate release dosage form with dose-proportional bioavailability and advantageous stability profiles where dosage forms preferably disintegrate rapidly.

The formulation of the present invention includes neramexane (1-amino-1,3,3,5,5-pentamethylcyclohexane), or an optical isomer, diastereomer, enantiomer, hydrate or pharmaceutically acceptable salt thereof, an optionally pharmaceutically acceptable coating, a pharmaceutically acceptable filler selected from microcrystalline cellulose, wherein the microcrystalline cellulose is present in a range from about 20% w/w to about 95% w/w and an additional disintegrant, but is free of lactose to be administered in a single oral dosage form, preferably once a day. Alternatively, the dosage form may be administered twice a day, with about 4 to about 8 hours between each administration. The dosage form is a tablet.

Specifically, the present invention provides a dosage form which immediately releases the active agent, neramexane, at a rate of about 80% or more within the first 60 minutes following entry of the dosage form into a use environment. Preferably, the dosage form is released to this extent within the first 30 minutes, more preferably, within the first 15 minutes.

In the present invention, the Tₘₐₓ for neramexane containing dosage forms is achieved at a time interval averaging from about 2 hours to about 8 hours after entry of the dosage form into the use environment with an active ingredient load of 2.5 to 150mg. Preferably, the time interval averages between about 3 to about 8 hours.

In specific embodiments where the active ingredient is neramexane mesylate, the active ingredient of the present invention is usually present in amounts ranging from about 2 % w/w to about 50% w/w. Preferably, the amounts range from about 2 % w/w to about 40 % w/w, more preferably from about 3 % w/w to about 25 % w/w.

In the present invention, the preferred optional pharmaceutically acceptable coating contains hydroxypropyl methylcellulose, such as Opadry^{®} (Colorcon, West Point, PA) or Sepifilm^{®} (Seppic, NJ) present in amounts ranging from about 2 % w/w to about 7 % w/w, preferably from about 2 % w/w to about 5 % w/w.

In appropriate embodiments, the formulation contains fillers such as starch and starch derivatives, hydrated sugar alcohols, calcium phosphates, and cellulose based excipients and derivatives thereof.

The oral dosage form of the present invention may further comprise one or more pharmaceutically acceptable carriers, excipients, anti-adherants, stabilizing agents, binders, colorants, disintegrants, glidants, and lubricants.

The dosage forms contain excipients that have improved stability, forming less than 3.0 % w/w lactose adduct, preferably less than 2.5 % w/w, upon storage for 36 months at room temperature. The present invention discovered the lactose adduct formation, which was not a foreseen adduct formation reaction. One skilled in art will recognize that an adduct, such as a lactose adduct, is formed by a Maillard reaction between the 1-aminocyclohexane analog active ingredient and a lactose excipient.

In the present invention, where no lactose (or any other reducing agent) is present, the microcrystalline cellulose filler is present in amounts ranging from about 20 % w/w to about 95 % w/w, preferably, in amounts ranging from about 60 % w/w to about 90 % w/w. Such dosage forms exhibit less than 0.5 % adduct formation in 36 months.

In another embodiment of the present invention, the dosage forms contain the lubricant magnesium stearate, which is present in amounts ranging from about 0 % to about 2% w/w, preferably, in amounts ranging from about 0.2 % to about 0.5 % w/w.

In another embodiment, the dosage forms contain an excipient which supports the disintegration of the formulation. This excipient may be starch-based or derivatives thereof, cellulose-based or derivatives thereof, or based on pyrrolidone or a derivative thereof, in amounts ranging from about 0.2 to 10 % w/w.

The composition is in tablet form. The tablet form has a hardness of from about 3 to about 40 Kp. Preferably, the hardness is from about 4 to about 30 Kp. One skilled in art will recognize that hardness of the tablet is also related to shape and size of tablets.

### DETAILED DESCRIPTION OF THE INVENTION

In accordance with the present invention, an immediate release pharmaceutical composition is provided for the administration of a 1-aminocyclohexane, neramexane, or an optical isomer, diastereomer, enantiomer, hydrate or pharmaceutically acceptable salt thereof, to a human or animal subject, where the composition includes oral solid dosage forms, in tablet form.

In the present invention, the pharmaceutical compositions comprise a therapeutically effective amount of neramexane (free base), or an optical isomer, diastereomer, enantiomer, hydrate or a pharmaceutically acceptable salt thereof, preferably the HCl salt and optionally a pharmaceutically acceptable coating, as well as, optionally, one or more carriers, fillers, anti-adherants, excipients, stabilizing agents, binders, colorants, disintegrants, glidants, and lubricants (all pharmaceutically acceptable).

Neramexane (1-amino-1,3,3,5,5-pentamethylcyclohexane) can be considered an analog of 1-amino-cyclohexane (disclosed, e.g., in U.S. Patent Nos. 4,122,193; 4,273,774; 5,061,703), and is a systemically-active noncompetitive NMDA receptor antagonist having low to moderate affinity for the receptor and strong voltage dependency and rapid blocking/unblocking kinetics. These pharmacological features allow neramexane to block sustained activation of the receptor under pathological conditions and to rapidly leave the NMDA channel during normal physiological activation of the channel.

The 1-aminocyclohexane compound used according to the invention is 1-amino-1,3,3,5,5-pentamethylcyclohexane (neramexane), its optical isomers, diastereomers, enantiomers, hydrates, and its pharmaceutically acceptable salts.

Neramexane (1-amino-1,3,3,5,5-pentamethylcyclohexane) is disclosed, in e.g., U.S. Patent No. 6,034,134.

According to the invention, neramexane may be applied as such or used in the form of its pharmaceutically acceptable salts. Suitable salts of the compound include, but are not limited to, acid addition salts, such as those made with hydrochloric, methylsulfonic, hydrobromic, hydroiodic, perchloric, sulfuric, nitric, phosphoric, acetic, propionic, glycolic, lactic pyruvic, malonic, succinic, maleic, fumaric, maleic, tartaric, citric, benzoic, carbonic cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid; salts made with saccharin. In a preferred embodiment, the salt is neramexane mesylate (C₁₁H₂₃N·CH₄O₃S, MW 265.42). The term "salts" can also include addition salts of free acids. All of these salts (or other similar salts) may be prepared by conventional means. All such salts are acceptable provided that they are non-toxic and do not substantially interfere with the desired pharmacological activity.

The present invention further includes all individual enantiomers, diastereomers, racemates, and other isomers of those compounds wherein such structural variations are possible. The invention also includes all polymorphs and solvates, such as hydrates and those formed with organic solvents, of these compounds. Such isomers, polymorphs, and solvates may be prepared by methods known in the art, such as by crystallization from different solvents, or by regiospecific and/or enantioselective synthesis and resolution, based on the disclosure provided herein.

The present invention includes derivatives of the compound of the present invention. Examples of derivatives applicable to the invention include, but are not limited to, structurally related compounds composed of a tricyclic 10-carbon ring bearing an amino group such as nitroxy-memantine derivatives (such as nitroprusside, nitroglycerin, or an NO-generating derivative of nitroprusside or nitroglycerin in U.S. Patent Nos. 5,234,956 and 5,455,279).

In a preferred embodiment, the active ingredient is neramexane mesylate. The active ingredient is present in amounts ranging broadly from about 6.25 mg to about 150 mg, preferably ranging from about 12.5 mg to about 125 mg. The active ingredient, e.g., neramexane mesylate in the oral dosage form of the present invention is usually present in amounts ranging from about 2 % w/w to about 50 % w/w. Preferably, the amounts range from about 2 % w/w to about 40 % w/w, more preferably from about 3 % w/w to about 25 % w/w.

The immediate-release dosage form optionally has a coating applied or deposited over the entire surface of a unitary release core. Immediate release of the drug is achieved by any of various methods known in the art including the use of a very thin layer or coating, which by virtue of its thinness (i.e., less than about 100 micron) is quickly penetrated by gastric fluid allowing fast leaching of the drug.

In the present invention, examples of coating materials that rapidly disintegrate and disperse include lactose and microcrystalline cellulose, colloidal silicon dioxide, hydrophilic polymers such as hydroxypropyl methylcellulose, PVA, methacrylates (*e.g*., Eudragit,® Rohm Pharma Polymer, Piscataway, NJ) natural polymers such as xanthan gum, and combinations thereof (*e.g.* Prosolv^{®}, which contains microcrystalline cellulose and colloidal silicone dioxide). In formulations with a lactose free environment, colloidal silicon dioxide may be necessary in addition to the use of microcrystalline cellulose, e.g., Avicel^{®}. These materials may also be present as excipients in addition to common auxiliary agents and additives or fillers including tabletting aids, colorants, binders, fillers, glidants, and lubricants (all pharmaceutically acceptable).

In one preferred embodiment of the invention, hydroxypropyl methylcellulose is used as a coating material. The optional coating material is present in amounts ranging from about 1 mg to about 70 mg, preferably from about 3 mg to about 60 mg, more preferably from about 3 mg to about 40 mg. In a preferred embodiment, compositions contain from about 2% w/w to about 5% w/w coating material containing hydroxypropyl methylcellulose; more preferably from about 2% to about 4% w/w coating material containing hydroxypropyl methylcellulose.

Fillers or disintegrants, act to modify the dissolution pattern. Examples of such fillers include lactose monohydrate, microcrystalline cellulose, Prosolv^{®}, hydroxypropyl methylcellulose, and combinations thereof. Lactose monohydrate, when used, counterbalances the less soluble ingredients of the composition, thereby acting as a disintengrant, whereas microcrystalline cellulose and similar type filler when employed in a lactose-free environment may require additional disintegrants such as croscarmellose sodium. Disintegrants in the dosage forms may further contain an excipient to support the disintegration of the formulation. One skilled in art recognizes that these excipients may be starch based, cellulose based or pyrrolidone based, or a derivative thereof, in amounts ranging from about 0.2 to 10 %.

When hydroxypropyl methylcellulose or ethyl cellulose are used in a matrix tablet, the dissolution rates are much lower than the immediate release rate targeted. This is because the hydrophobic matrix tablets that result when these polymers release the drug by mechanism of polymer erosion. Since the erosion from a hydrophobic matrix is very slow, the dissolution rate of the readily soluble active ingredient is also slow.

In the dosage forms of the invention, microcrystalline cellulose (MCC) is used as a filler in the absence of lactose monohydrate. The MCC is present in an amount ranging from about 50 mg to about 1,600 mg, preferably from about 100 mg to about 1,200 mg per unit dose. The compositions contain from about 20 % w/w to about 95 % w/w microcrystalline cellulose; more preferably from about 60 % w/w to about 90 % w/w. The microcrystalline cellulose provides the desired dissolution profiles with acceptable or improved formulation and processing properties. One skilled in art will recognize that these microcrystalline cellulose based formulations contain disintegrants. Disintgrants are starch-based, cellulose-based or pyrrolidone-based excipients, or based on a derivative of any of the foregoing, in amounts ranging from about 0.2 to 10 % w/w.

Additional excipients such as talc (an anti-adherant), starch, dicalcium phosphate, mannitol, croscarmellose sodium, colloidal silicon dioxide, sodium starch glycolate can also be used in combination. Use of the disinetgrants or soluble fillers allow for rapid disintegration of tablets exposing large surface area and the drug leading to faster dissolution of the drug.

Additionally, the dosage forms contain excipients that form less than 3.0 % adduct, preferably less than 2.5%, even 0% in lactose-free formulations. One skilled in art will recognize that substances such as memantine and neramexane adducts result from a Maillad reaction. Adducts, such as the lactose or other reducing sugar adducts, may form with the amines in adamantane derivatives.

Tablets in accordance with this invention can be prepared by conventional mixing, comminution, and tabletting techniques that are well-known in the pharmaceutical formulations industry. The immediate-release tablet, for example, may be fabricated by direct compression by punches and dies fitted to a rotary tabletting press, ejection or compression molding, granulation followed by compression, or forming a paste and extruding the paste into a mold or cutting the extrudate into short lengths followed by compression. As mentioned above, the immediate release component may be applied as a coating over the core by spraying, dipping, or pan-coating, or as an additional layer by tabletting or compression. Preferably, the process used for preparing tablets is direct compression of the blend. Ordinarily, direct blending is a difficult process, and problems such as blend segregation, low compressibility and low content uniformity can occur. However, neither the formulations described in this invention nor the process for making them exhibit these problems, or such problems are substantially less significant. Near IR spectroscopic methods showed good distribution of the drug in the tablets.

When tablets are made by direct compression, the addition of lubricants may be helpful and is sometimes important to promote powder flow and to prevent "capping" of the tablet (the breaking off of a portion of the tablet) when the pressure is relieved. Useful lubricants are magnesium stearate and hydrogenated vegetable oil (preferably hydrogenated and refined triglycerides of stearic and palmitic acids). In a preferred embodiment, magnesium stearate is used as a lubricant in an amount from about 0 mg to about 6 mg, preferably from about 0.3 mg to about 4.0 mg. In a preferred embodiment, the compositions contain from about 0 % w/w to about 2 % w/w magnesium stearate; more preferably from about 0.2 % w/w to about 0.5 % w/w magnesium stearate. Additional excipients may be added to enhance tablet hardness, powder flowability, and to reduce tablet friability and adherence to the die wall.

Tablet hardness is linearly affected by different compression forces, shape and size of the tablet. As compression forces increase (kN), there is a linear increase in tablet hardness (Kp). Preferably, hardness values range from about 3 to about 40 Kp, more preferably from about 4 to about 30 Kp. In addition, at lower compression, and thus lower hardness values, e.g., lower than 3 Kp, the logo and product identification de-bossing was "picked" making it difficult to read and aesthetically less pleasing. At the higher compression and hardness values, the picking was eliminated without affecting dissolution at 30 minutes.

The plasma concentration of the dose proportional immediate release formulations of neramexane have a time of maximum plasma concentration (Tₘₐₓ) ranging from between about 2 to about 8 hours, more often averaging between about 2 to about 7 hours, and an in vitro release rate of more than about 80 % in about 60 minutes, more preferably in about 30 minutes.

The pharmaceutical formulations of the present invention allow for dose-proportional compositions and the modification of the Cₘₐₓ by changing the strength of the formulation without substantially affecting the Tₘₐₓ of the drug. The 30-minute immediate release formulations described in the present invention provide the desired Tₘₐₓ without compromising the initial peak (Cₘₐₓ), which is characteristic of memantine or neramexane salts.

In addition, a long T_{½} allows for either twice a day, or preferably once a day, administration for an immediate release dosage form and achieves a relatively high Cₘₐₓ which is considered essential for the pharmacological efficacy of the product. If the neramexane dosage form is administered twice a day, administration being approximately 4 hours apart, the average Tₘₐₓ is about 8 hours ± 2 hours. In addition, the dose proportionality allows up-titration beginning with lower doses for patient using an essentially identical formulation composition and varying essentially only the weight content of memantine or neramexane to achieve different strengths.

In accordance with the present invention, an immediate release pharmaceutical composition is provided for the once daily administration or, if preferred, twice per day, of neramexane or an optical isomer, diastereomer, enantiomer, hydrate or a pharmaceutically acceptable salt thereof, preferably its mesylate salt, to a human or animal subject.

In an alternative embodiment of the invention, the rapid dissolution profile of the tablets enables drinkable solutions for patients unable to ingest tablets.

The neramexane formulations of the invention are suitable for the treatment of CNS diseases, including but not limited to the treatment of Alzheimer's disease, Parkinson's disease, AIDS dementia (U.S. Patent No. 5,506,231, see also Parsons et al., Neuropharmacology 1999 Jun;38(6):735-67), neuropathic pain (U.S. Patent No. 5,334,618), cerebral ischemia, epilepsy, glaucoma, hepatic encephalopathy, multiple sclerosis, stroke, depression (U.S. Patent No. 6,479,553), tardive dyskinesia, malaria, Borna virus, Hepatitis C (U.S. Patent Nos. 6,034,134 and 6,071,966). Additional pathologies for treatment of which memantine is suitable are disclosed in U.S. Patent Nos. 5,614,560 and 6,444,702. Accordingly, the present invention further provides a method for the therapeutic or prophylactic treatment of CNS disorders in a human or animal subject, the method including administering to the subject a composition in accordance with the present invention.

As used herein, "adduct formation" refers to the formation of a compound with a particular formulation of a composition by a solid phase reaction. The general term "adduct" for a compound, also called an addition compound, results from the direct combination of two or more different compounds. For example, in the present invention, lactose adduct formation may occur with formulations containing lactose. Such adduct formation detracts from the efficacy of the product and increases the risks of other side effects.

As used herein, a "therapeutically effective amount" means the amount of a compound that, when administered to a mammal for treating a state, disorder or condition is sufficient to effect such treatment. The "therapeutically effective amount" will vary depending on the compound, the disease and its severity and the age, weight, physical condition and responsiveness of the mammal to be treated. According to the present invention, in one embodiment, a therapeutically effective amount of memantine is an amount effective to treat CNS disorders, including Alzheimer's disease or Parkinson's disease. Other uses include, but are not limited to, the treatment of dementia and depression. The effective amount of the drug for pharmacological action, and therefore the tablet strength, depends on the disease itself, *e.g*., in Alzheimer's disease, the patient is initially given a 5 mg dose and the dosage is progressively increased to 10 mg twice a day to 20 mg once a day. Similar up-titrations but starting from higher base amounts (e.g., base values starting at about 12 to about 15 mg, titrating up to about 80 mg) are useful for pain relief, e.g., neuropathic pain. Such titration may be facilitated by providing a selection of tablets representing standard or common doses, for example, 5mg, 10 mg, 15 mg, 20 mg, 40 mg and 80 mg doses of active substance. Therefore, it is important to have a dose proportional formulation.

As used herein, the term "pharmaceutically acceptable" refers to biologically or pharmacologically compatible for *in vivo* use, and preferably means approved by a regulatory agency of the Federal or a state government or listed in the U.S. Pharmacopeia or other generally recognized pharmacopeia for use in animals, and more particularly in humans.

As used herein, the term "treat" and its derivatives are used herein to mean to relieve or alleviate pain in a hypersensitive mammal or in a mammal suffering from a CNS disorder, e.g., dementia or Parkinson's disease. The term "treat" may mean to relieve or alleviate the intensity and/or duration of a manifestation of disease experienced by a subject in response to a given stimulus (*e.g*., pressure, tissue injury, cold temperature, etc.). For example, in relation to dementia, the term "treat" may mean to relieve or alleviate cognitive impairment (such as impairment of memory and/or orientation) or impairment of global functioning (activities of daily living, ADL) and/or slow down or reverse the progressive deterioration in ADL or cognitive impairment. Within the meaning of the present invention, the term "treat" also denotes to arrest, delay the onset (*i.e*., the period prior to clinical manifestation of a disease) and/or reduce the risk of developing or worsening a disease. The term "protect" is used herein to mean prevent delay or treat, or all, as appropriate, development or continuance or aggravation of a disease in a subject. Within the meaning of the present invention, the dementia is associated with a CNS disorder, including without limitation neurodegenerative diseases such as Alzheimer's disease (AD).

The term "picking" refers to the detachment of material (such as a film fragment) from the surface of a tablet upon contact with another object and its adherence to the surface of the other object (such as another tablet or a tooling) (See Pharmaceutical Dosage Forms: Tablets Volume 3, edited by H. A. Lieberman and L. Lachman, pp. 101 and 272 (Marcel Dekker, Inc. 1982)). Picking may occur, for example, when tablets are compressed or tumbled. The material removed may obscure or obliterate logos, monograms, lettering, and numbering which were intended to appear on the surface of the tablet.

The term "dose proportional" as used herein refers to the relationship between the dose of a drug and its bioavailability. For example, in the present invention, twice as much of the same composition to make a dosage form that will deliver twice the drug will provide the same bioavailability (i.e., AUC and Cₘₐₓ) as one dose of the dosage form. The dose proportionality of the present invention applies to a wide range of doses as discussed in detail herein.

The term "about" or "approximately" means within an acceptable error range for the particular value as determined by one of ordinary skill in the art, which will depend in part on how the value is measured or determined, *i.e*., the limitations of the measurement system. For example, "about" can mean within 1 or more than 1 standard deviations, per practice in the art. Alternatively, "about" with respect to the compositions can mean plus or minus a range of up to 20%, preferably up to 10%, more preferably up to 5%. Alternatively, particularly with respect to biological systems or processes, the term can mean within an order of magnitude, preferably within 5-fold, and more preferably within 2-fold, of a value. Where particular values are described in the application and claims, unless otherwise stated the term "about" means within an acceptable error range for the particular value. For example, when referring to a period of time, e.g., hours, the present values (± 20%) are more applicable. Thus, 6 hours can be, e.g., 4.8 hours, 5.5 hours, 6.5 hours, 7.2 hours, as well as the usual 6 hours.

The term "use environment" when applied to the formulations means the gastric fluids of a patient to whom the formulation is administered or simulated dissolution media.

### EXAMPLES

The present invention will be better understood by reference to the following Examples, which are provided as exemplary of the invention, and not by way of limitation.

### EXAMPLE 1: Preparation of Immediate Release Tablets

### Materials and Methods

The following tables provide the makeup of immediate release tablets including the active components, coating agent, and other excipients for the specified dosage forms with specific target release time periods.

Tables 1 and 2 provide the makeup of tablets without lactose and contain the same data expressed respectively in absolute (mg) or relative (% w/w) terms.

**Table 1. 6.25 mg to 125 mg Dose Proportional Formulations (lactose free) Exact formula composition (Composition in mg per Tablet)**

| Excipient | **6.25 mg** | **12.5mg** | **25 mg** | **37.5 mg** | **50 mg** | **75 mg** | **100 mg** | **125 mg** |
|---|---|---|---|---|---|---|---|---|
| Neramexane Mesylate | 6.25 | 12.5 | 25.0 | 37.5 | 50.0 | 75.0 | 100.0 | 125.0 |
| Microcrystalline Cellulose (Avicel® or ProSolv®)* | 51.6 | 103.2 | 206.5 | 309.7 | 413.0 | 619.5 | 826.0 | 1032.5 |
| Colloidal Silicon Dioxide | 0.6 | 1.3 | 2.5 | 3.8 | 5.0 | 7.5 | 10.0 | 12.5 |
| Croscarmellose Sodium | 3.1 | 6.3 | 12.5 | 18.8 | 25.0 | 37.5 | 50.0 | 62.5 |
| Talc | 0.6 | 1.3 | 2.5 | 3.8 | 5.0 | 7.5 | 10.0 | 12.5 |
| Magnesium Stearate | 0.2 | 0.5 | 1.0 | 1.5 | 2.0 | 3.0 | 4.0 | 5.0 |
| Total core tablet* | 62.5 | 125.0 | 250.0 | 375.0 | 500.0 | 750.0 | 1000.0 | 1250.0 |
| Coating (HPMC), Opadry or Sepifilm | 2.5 | 5.0 | 10.0 | 15.0 | 20.0 | 30.0 | 40.0 | 50.0 |
| Total coated | 65 | 130 | 260 | 390 | 520 | 780 | 1040 | 1300 |

| | | | | | | | | |
|---|---|---|---|---|---|---|---|---|
| *Core weight may be adjusted with fillers to +/- 10% depending on filler density. | | | | | | | | |

For the dose proportional formulations of Table 1, the percentage w/w for each of the active ingredient and excipients are identified in Table 2.

**Table 2. Weights in % w/w of tablet (lactose free) all strengths, including high drug load)**

| Excipient | | Neramexane Tablets (6.25-150 mg) |
|---|---|---|
| Memantine Hydrochloride | | 0 |
| Neramexane Mesylate | | 9.6 |
| Microcrystalline Cellulose (Prosolvo^{®}, or Avicel® plus Colloidal Silicon Dioxide) | | 79.4 |
| Colloidal Silicon Dioxide | | 1.0 (Avicel®) |
| Croscarmellose Sodium | | 4.8 |
| Talc | | 1.0 |
| Magnesium Stearate | | 0.4 |
| Coating (HPMC), Opadry or Sepifilm | | 3.8 |
| Total | | 100.0% |

Test batches of each of the tablets were prepared according to the process outlined below.

*Preparation of Blend for Tabletting (lactose*/*MCC).* Approximately half of the amount of microcrystalline cellulose and active drug was placed into a 20 ft³ cone blender. Colloidal silicon dioxide was screened with the remainder of the microcrystalline cellulose through about 0.71 mm screen and added to the 20 ft³ cone blender. The components were mixed for 6 minutes with the intensifier bar off. The lactose monohydrate (when called in the formula) and talc were screened through about 0.71 mm and added to the cone blender. The blender contents were mixed for 20 minutes with the intensifier bar off. The magnesium stearate was screened through about 0.8 mm filter and was added to the cone blender. The mixture was blended for an additional five minutes with the intensifier bar off. One skilled in art will recognize that for the MCC and other fillers, the above process may be modified. One skilled in art will recognize that alternated addition and mixing methods are also acceptable.

During the process of manufacturing the tablets, before the compression into tablet form, an initial batch of blended product was blended for 2 hours, with samples obtained throughout the time period. The samples were tested for segregation.

*Compression of Tablets.* The blend was compressed using a rotary tablet press. Tablets were compressed at different compression forces ranging from 5 to 25 Kp and tested for physical properties hardness, dissolution, thickness, friability, and content uniformity. For dissolution tests, tablets of different hardness were tested using USP Apparatus II using 900 ml of pH 1.2 buffer. The tablets were passed through a tablet deduster and metal checker after compression. The tablets were then coated in a perforated coating pan.

Tests were also conducted to study the effect of coating on dissolution and stability. Tablets were coated with Opadry (containing hydroxypropyl methylcellulose) material. A dissolution testing apparatus at 100 rpm was used to generate results. Alternate dissolution methods, e.g. 50 rpm using appropriate USP apparatus is also acceptable. Samples were collected after various levels of weight gain (based on amount of coating) and tested for dissolution at 15, 30, and 45 minutes. To determine the stability, coated tablets were put in a chamber under 40°C/75%RH accelerated conditions in an open dish for three months. Dissolution testing was carried out at 15, 30, and 45 minutes.

### Results and Discussion

The samples obtained during the 2-hour blending test exhibited no noticeable deblending. The results showed that the formula ingredients allowed for good distribution of the active ingredients and that, once blended, the active ingredient remained uniformly distributed throughout the tablet matrix. The mixing time of 20 minutes (400 revolutions) was chosen as the preferred blending time. A lack of significant shifts in particle size distribution were observed regardless of blend time, indicating that no measurable particle attrition took place during blending. The results were well within the limits of the USP content uniformity test for tablets.

The results of the effect of compression force on tablet hardness showed that as compression force (kN) increased, a linear increase in tablet hardness (Kp) also occurred. Similarly, as compression force increased, there was a linear decrease in tablet thickness (inches). One unfavorable development during compression was the appearance of tablet sticking. Lower punches were embossed with the tablet strength (5, 10 or 20) and upper punches with "FP". Sticking to the punches, particularly the "P", was observed at lower compression forces. Producing harder tablets eliminated the sticking issue.

The effect of tablet hardness on dissolution was evaluated further. The data showed that hardness has an effect on dissolution. This effect was only observed for the 15 minutes time point, which relates to the disintegration of the tablets. Complete release was obtained for the 30 minutes time point. The proposed dissolution specification for the product was no less than 80% dissolved in 30 minutes. Based on the data, the higher tablet hardness required to avoid sticking will have no effect on the dissolution specification. The data for hardness and dissolution values is present in Tables 3 and 4 below.

**Table 3. Dissolution of Neramexane Mesylate Core Tablets of different hardness (Filler lactose-free)**

| | | | | | | |
|---|---|---|---|---|---|---|
| Strength mg | 12.5 mg | | 25 mg | | 50 mg | |
| Lot # RD- | 0943-1B | | 0903-144A | | 0903-144C | |
| Hardness (Kp) | 6kp | 11kp | 13kp | 22 kp | 21kp | 35kp |

| Time (min) | % Dissolved | | | | | |
|---|---|---|---|---|---|---|
| 15 | 105 | 104 | 96 | 96 | 101 | 99 |
| 30 | 106 | 107 | 101 | 99 | 109 | 103 |
| 60 | 102 | 105 | 101 | 100 | 110 | 107 |

**Table 4. Dissolution of Neramexane Mesylate Coated Tablets**

| | | |
|---|---|---|
| Strength | 12.5 mg | 25 mg |
| Lot # RD- | 1033-29A | 1033-4A |
| Core table hardness (Kp) | 7-9 | 16-18 |

| % Dissolved | | |
|---|---|---|
| 15 min | 100 | 103 |
| 30 min | 102 | 103 |
| 60 min | 102 | 103 |

Tablet friability was tested since the product was film-coated to mask the characteristic taste of the drug. Generally, the friability values were very low, indicating good mechanical integrity for the tablets. Tablet content was reviewed for uniformity, and in all cases tablets had low variability in content.

Initial dissolution testing was also conducted. A target dissolution of no less than 80% in 30 minutes was desired in order to support a Biopharmaceutical Classification System (BCS) Class 1 classification for the drug. Tablets also showed rapid dissolution (greater than 80% in 30 minutes) even at very high hardness (20 Kp for 20 mg tablets).

Study results also showed that the coating process and the coating level had no effect on dissolution and stability of the final products. No significant changes were observed after three months under the extreme conditions, demonstrating the stability of the formulations. The dry blend process designed is very resistant to blend segregation and it is not sensitive to particle size distribution of the active or blend. The tablets showed good mechanical integrity (with compression force of 10 kN for 5 mg tablets) and good content uniformity.

It is further to be understood that all values are approximate, and are provided for description.

## Claims

1. An immediate release solid oral dosage form comprising (i) an active ingredient which is neramexane or an optical isomer, diastereomer, enantiomer, hydrate, or pharmaceutically acceptable salt thereof, (ii) a pharmaceutically acceptable filler selected from microcrystalline cellulose, wherein the microcrystalline cellulose is present in a range from about 20% w/w to about 95% w/w, and (iii) an additional distintegrant, said dosage form exhibiting dose-proportionality, releasing said active ingredient at a rate of more than 80 % within about the first 60 minutes following entry of said form into a use environment, and exhibiting an average Tₘₐₓ of 2 to 8 hours with an active ingredient load of 2.5 to 150 mg, wherein said dosage form is free of lactose and wherein said dosage form is a tablet.

2. A dosage form according to claim 1 which releases said active ingredient at a rate of more than 80% within the first 30 minutes following entry into a use environment, preferably at a rate of more than 80% within the first 15 minutes following entry of into a use environment.

3. A dosage form according to any preceding claim, wherein said active ingredient is neramexane mesylate.

4. A dosage form according to claim 3, wherein the neramexane mesylate is present in an amount of 2 to 50% w/w, preferably 2 to 40% w/w, even more preferably 3 to 25 % w/w.

5. A dosage form according to any preceding claim further comprising a pharmaceutically acceptable coating.

6. A dosage form according to claim 5, wherein the pharmaceutically acceptable coating contains hydroxypropyl methylcellulose or a methacrylic acid-ethyl acrylate copolymer.

7. A dosage form according to claim 5 or claim 6, wherein the pharmaceutically acceptable coating is present in an amount of 2 to 7% w/w, preferably 2 to 5% w/w.

8. A dosage form according to any preceding claim, further comprising one or more pharmaceutically acceptable carriers, excipients, anti-adherents, fillers, stabilizing agents, binders, colorants, disintegrants, glidants, and lubricants.

9. A dosage form according to any preceding claim, wherein the microcrystalline cellulose is present in an amount of 60 to 90 % w/w.

10. A dosage form according to any preceding claim which has a hardness of 3 to 40 Kp, preferably 4 to 30 Kp.

11. A dosage form according to any of claims 8 to 10, wherein the lubricant is magnesium stearate, wherein the magnesium stearate is preferably present in an amount of up to 2% w/w, even more preferably 0.2 to 0.5 % w/w.

12. A dosage form according to any preceding claim for use in treating a disorder selected from the group consisting of mild, moderate, and severe Alzheimer's dementia, Parkinson's disease, AIDS dementia, neuropathic pain, cerebral ischemia, epilepsy, glaucoma, hepatic encephalopathy, multiple sclerosis, stroke, depression, tardive dyskinesia, malaria, Boma virus, and Hepatitis C.

13. Use of the immediate release solid oral dosage form of claim 1 for the manufacture of a medicament for the treatment of a disorder selected from the group consisting of mild, moderate, and severe Alzheimer's dementia, Parkinson's disease, AIDS dementia, neuropathic pain, cerebral ischemia, epilepsy, glaucoma, hepatic encephalopathy, multiple sclerosis, stroke, depression, tardive dyskinesia, malaria, Boma virus, and Hepatitis C.

14. A method for the manufacture of a dosage form as claimed in any of claims 1 to 12 comprising direct compression.

## Patentansprüche

1. Unmittelbar freisetzende, feste orale Dosierungsform, umfassend (i) einen Wirkstoff, der Neramexan oder ein optisches Isomer, Diastereomer, Enantiomer, Hydrat oder pharmazeutisch verträgliches Salz davon ist, (ii) ein pharmazeutisch verträgliches Füllmaterial, das ausgewählt ist aus mikrokristalliner Zellulose, wobei die mikrokristalline Zellulose in einem Bereich von ungefähr 20 Gew.-% bis ungefähr 95 Gew.-% vorhanden ist, und (iii) ein zusätzliches Zerfallsmittel, wobei die Dosierungsform Dosisproportionalität aufweist, welche den Wirkstoff mit einer Geschwindigkeit von mehr als 80 % innerhalb ungefähr der ersten 60 Minuten im Anschluss an den Eintritt der Form in eine Anwendungsumgebung freisetzt und einen durchschnittlichen Tₘₐₓ von 2 bis 8 Stunden mit einer Wirkstoffbeladung von 2,5 bis 150 mg aufweist, wobei die Dosierungsform frei von Laktose ist und wobei die Dosierungsform eine Tablette ist.

2. Dosierungsform nach Anspruch 1, welche den Wirkstoff mit einer Geschwindigkeit von mehr als 80 % innerhalb der ersten 30 Minuten im Anschluss an den Eintritt in eine Anwendungsumgebung freisetzt, vorzugsweise mit einer Geschwindigkeit von mehr als 80 % innerhalb der ersten 15 Minuten im Anschluss an den Eintritt in eine Anwendungsumgebung.

3. Dosierungsform nach einem der vorhergehenden Ansprüche, wobei der Wirkstoff Neramexan-Mesylat ist.

4. Dosierungsform nach Anspruch 3, wobei Neramexan-Mesylat in einer Menge von 2 bis 50 Gew.-%, vorzugsweise von 2 bis 40 Gew.-%, noch mehr bevorzugt von 3 bis 25 Gew.-% vorhanden ist.

5. Dosierungsform nach einem der vorhergehenden Ansprüche, die ferner eine pharmazeutisch verträgliche Beschichtung umfasst.

6. Dosierungsform nach Anspruch 5, wobei die pharmazeutisch verträgliche Beschichtung Hydroxypropylmethylcellulose oder ein Methacrylsäure-Ethylacrylat-Copolymer enthält.

7. Dosierungsform nach Anspruch 5 oder Anspruch 6, wobei die pharmazeutisch verträgliche Beschichtung in einer Menge von 2 bis 7 Gew.-%, vorzugsweise von 2 bis 5 Gew.-% vorliegt.

8. Dosierungsform nach einem der vorhergehenden Ansprüche, die ferner einen oder mehrere pharmazeutisch verträgliche Trägerstoffe, Hilfsstoffe, Antihaftmittel, Füllstoffe, Stabilisierungsmittel, Bindemittel, Färbemittel, Zerfallsmittel, Gleitmittel und Schmiermittel umfasst.

9. Dosierungsform nach einem der vorhergehenden Ansprüche, wobei die mikrokristalline Cellulose in einer Menge von 60 bis 90 Gew.-% vorliegt.

10. Dosierungsform nach einem der vorhergehenden Ansprüche, die eine Härte von 3 bis 40 Kp, vorzugsweise 4 bis 30 Kp aufweist.

11. Dosierungsform nach einem der Ansprüche 8 bis 10, wobei das Schmiermittel Magnesiumstearat ist, wobei das Magnesiumstearat vorzugsweise in einer Menge von bis zu 2 Gew.-%, sogar noch bevorzugter von 0,2 bis 0,5 Gew.-% vorliegt.

12. Dosierungsform nach einem der vorhergehenden Ansprüche zur Verwendung in der Behandlung einer Erkrankung, die ausgewählt ist aus der Gruppe, die aus leichter, mittelschwerer und schwerer Alzheimer'scher Demenz, Parkinson-Erkrankung, AIDS-Demenz, neuropathischen Schmerzen, zerebraler Ischämie, Epilepsie, Glaukom, hepatischer Enzephalopathie, Multipler Sklerose, Schlaganfall, Depression, tardiver Dyskinesie, Malaria, Borna-Virus und Hepatitis C besteht.

13. Verwendung der unmittelbar freisetzenden, festen oralen Dosierungsform nach Anspruch 1 für die Herstellung eines Medikaments zur Behandlung einer Erkrankung, die ausgewählt ist aus der Gruppe, die aus leichter, mittelschwerer und schwerer Alzheimer'scher Demenz, Parkinson-Erkrankung, AIDS-Demenz, neuropathischen Schmerzen, zerebraler Ischämie, Epilepsie, Glaukom, hepatischer Enzephalopathie, Multipler Sklerose, Schlaganfall, Depression, tardiver Dyskinesie, Malaria, Borna-Virus und Hepatitis C besteht.

14. Verfahren zur Herstellung einer Dosierungsform nach einem der Ansprüche 1 bis 12, welches die direkte Komprimierung umfasst.

## Revendications

1. Forme galénique solide orale à libération immédiate comprenant (i) un principe actif qui est le néramexane ou un isomère optique, un diastéréomère, un énantiomère, un hydrate, ou un sel pharmaceutiquement acceptable de celui-ci, (ii) un agent de remplissage pharmaceutiquement acceptable choisi parmi la cellulose microcristalline, la cellulose microcristalline étant présente dans une plage d'environ 20 % p/p à environ 95 % p/p, et (iii) un agent de délitement supplémentaire, ladite forme galénique présentant une proportionnalité de la dose, libérant ledit principe actif à un taux de plus de 80 % durant à peu près les 60 premières minutes à la suite de la pénétration de ladite forme dans un environnement d'utilisation, et présentant un Tₘₐₓ moyen de 2 à 8 heures avec une charge en principe actif de 2,5 à 150 mg, ladite forme galénique étant dépourvue de lactose et ladite forme galénique étant un comprimé.

2. Forme galénique selon la revendication 1 qui libère ledit principe actif à un taux de plus de 80 % durant à peu près les 30 premières minutes à la suite de la pénétration dans un environnement d'utilisation, de préférence à un taux de plus de 80 % durant les 15 premières minutes à la suite de la pénétration dans un environnement d'utilisation.

3. Forme galénique selon l'une quelconque des revendications précédentes, dans laquelle ledit principe actif est le mésylate de néramexane.

4. Forme galénique selon la revendication 3, dans laquelle le mésylate de néramexane est présent dans une quantité de 2 à 50 % p/p, de préférence 2 à 40 % p/p, de manière même davantage préférée 3 à 25 % p/p.

5. Forme galénique selon l'une quelconque des revendications précédentes, comprenant en outre un enrobage pharmaceutiquement acceptable.

6. Forme galénique selon la revendication 5, dans laquelle l'enrobage pharmaceutiquement acceptable contient de l'hydroxypropylméthylcellulose ou un copolymère d'acide méthacrylique-acrylate d'éthyle.

7. Forme galénique selon la revendication 5 ou la revendication 6, dans laquelle l'enrobage pharmaceutiquement acceptable est présent dans une quantité de 2 à 7 % p/p, de préférence 2 à 5 % p/p.

8. Forme galénique selon l'une quelconque des revendications précédentes, comprenant en outre un ou plusieurs supports, excipients, agents anti-adhérents, agents de remplissage, agents de stabilisation, liants, colorants, agents de délitement, agents de glissement, et lubrifiants.

9. Forme galénique selon l'une quelconque des revendications précédentes, dans laquelle la cellulose microcristalline est présente dans une quantité de 60 à 90 % p/p.

10. Forme galénique selon l'une quelconque des revendications précédentes, qui présente une dureté de 3 à 40 Kp, de préférence 4 à 30 Kp.

11. Forme galénique selon l'une quelconque des revendications 8 à 10, dans laquelle le lubrifiant est le stéarate de magnésium, le stéarate de magnésium étant présent dans une quantité allant jusqu'à 2 % p/p, de manière même davantage préférée 0,2 à 0,5 % p/p.

12. Forme galénique selon l'une quelconque des revendications précédentes, pour une utilisation dans le traitement d'un trouble choisi dans le groupe constitué de la démence d'Alzheimer légère, modérée et sévère, la maladie de Parkinson, la démence du SIDA, la douleur neuropathique, l'ischémie cérébrale, l'épilepsie, le glaucome, l'encéphalopathie hépatique, la sclérose en plaques, l'accident vasculaire cérébral, la dépression, la dyskinésie tardive, le paludisme, le virus de la maladie de Borna, et l'hépatite C.

13. Utilisation de la forme galénique solide orale à libération immédiate selon la revendication 1, pour la fabrication d'un médicament destiné au traitement d'un trouble choisi dans le groupe constitué de la démence d'Alzheimer légère, modérée et sévère, la maladie de Parkinson, la démence du SIDA, la douleur neuropathique, l'ischémie cérébrale, l'épilepsie, le glaucome, l'encéphalopathie hépatique, la sclérose en plaques, l'accident vasculaire cérébral, la dépression, la dyskinésie tardive, le paludisme, le virus de la maladie de Borna, et l'hépatite C.

14. Procédé de fabrication d'une forme galénique selon l'une quelconque des revendications 1 à 12, comprenant une compression directe.
